# EUROPEAN PATENT SPECIFICATION

(11) **EP 0 979 093 B1**
(45) Date of publication and mention of the grant of the patent: **19.12.2001**
(21) Application number: 98907023.0
(22) Date of filing: 24.02.1998
(51) Int. Cl.: A61K 35/76, C12N 7/01, C12N 15/86, A61K 48/00

(54) **ARRESTABLE THERAPEUTIC VIRAL AGENT**
THERAPEUTISCHER VIRALER WIRKSTOFF, WELCHER DAS FORTSCHREITEN VON TUMOREN STOPPT
AGENT VIRAL THERAPEUTIQUE STOPPANT LE DEVELOPPEMENT DE TUMEURS

(30) Priority: 27.02.1997 GB 9704046
(43) Date of publication of application: 16.02.2000
(73) Proprietor: THE UNIVERSITY OF LEEDS, Leeds, LS2 9JT (GB)
(72) Inventor: MARKHAM, Alexander Fred, The University of Leeds, Leeds LS9 7TF (GB); MEREDITH, David Mark, The University of Leeds, Leeds LS9 7TF (GB)
(74) Representative: Harrison Goddard Foote
(86) International application number: GB9800559
(87) International publication number: WO9837905

(56) References cited:
- EP-A- 0 668 355
- WO-A-96/00007
- WO-A-97/04804
- WO-A-98/27216
- T. MINETA ET AL.: "ATTENUATED MULTI-MUTATED HERPES SIMPLEX VIRUS-1 FOR THE TREATMENT OF MALIGNANT GLIOMAS." NATURE MEDICINE., vol. 1, no. 9, September 1995, NEW YORK, NY., US, pages 938-943, XP002067917
- T. MINETA ET AL.: "TREATMENT OF MALIGNANT GLIOMAS USING GANGCICLOVIR-HYPERSENSITIVE, RIBONUCLEOTIDE REDUCTASE-DEFICIENT HERPES SIMPLEX VIRAL MUTANT." CANCER RESEARCH, vol. 54, 1 August 1994, BALTIMORE, MD, US, pages 3963-3966, XP002067918
- J.M. MARKERT ET AL.: "REDUCTION AND ELIMINATION OF ENCEPHALITIS IN AN EXPERIMENTAL GLIOMATHERAPY MODEL WITH ATTENUATED HERPES SIMPLEX MUTANTS THAT RETAIN SUSCEPTIBILITY TO ACYCLOVIR" NEUROSURGERY, vol. 32, no. 4, 1 April 1993, BALTIMORE, MD, US, pages 597-603, XP000561128

## Description

The invention relates to a method of virus manipulation; means therefor and products thereof which have particular, but not exclusive, application in therapy/vaccine development.

Current approaches to cancer therapy rely on the surgical removal, immunological destruction or targeted cytotoxicity of tumour tissue and/or cells. Most procedures currently in use are either physically invasive or have toxic side effects. A new form of therapy which is capable of reducing tumour mass without these problems would make a major impact on all aspects of cancer therapy.

Many animal viruses replicate in tissue culture cell lines derived from human tumours, with the concomitant destruction of the cultured cells. These cell lines may often be derived from cell types which would not normally support the replication of the virus *in vivo.* The difference is often due to the high metabolic activity of the cell line, in comparison with the resting or post-mitotic form which the virus might encounter *in vivo.* Furthermore tumour cells often demonstrate less regulated transcription patterns which may assist virus replication through the provision of transcriptional control proteins which may not normally exist in the differentiated cell.

Whereas these observations have accumulated in the scientific literature, viruses have not, until recently, been considered of use in cancer therapy, mainly because of the fear that virus replication would be uncontrolled and thus become potentially lethal to this host.

Retrovirus vectors which notably are replication-defective, and which express the human tumour suppressor gene P53 have been demonstrated to have efficacy in the therapy of primary lung cancer, causing either a halt in tumour growth or reduction in tumour size (1). These viruses halt the rapid growth of the tumour and presumably make it more susceptible to either committing programmed cell death (apoptosis) or stopping cell division.

Unfortunately many patients present with tumours which are well advanced, and are suffering secondary effects of tumour burden. Attempts are currently underway to develop a virus vector for the destruction of brain tumours, by the deletion of genes responsible for neuropathogenesis (2). Such viruses show promise in the selective destruction of rapidly growing tumour cells, whilst not replicating in normal neural tissue.

A major problem with the replication-competent viruses is that they may continue to grow in the patient after tumour destruction and cause other disease symptoms, particularly if they are derived from relatively low passage, virulent virus strains. Furthermore, the innate host immunity to these viruses may inhibit virus spread and destroy the infected tumour cells.

We have, contrary to prevailing wisdom and generally accepted practices in the art of cancer therapy, used replication-competent animal viruses in a most elegant way. We have exploited the phenomenon that all herpesviruses are variably susceptible to anti-herpetic drugs/agents as the premise of the invention. Additionally we have manipulated viruses in a unique manner so as to destroy/delete/deactivate selected genes associated with pathogenicity whilst optionally manipulating said viruses to achieve enhanced sensitivity to anti-herpetic drugs/agents. Consequently we have advantageously produced a replication-competent animal virus that is apathogenic to man and that can easily be arrested, either temporarily or permanently, at any time in the course of therapy.

Furthermore, anti-herpetic drugs are known to have negligible side effects even with repeated and long term dosing, and certainly represent a class of drugs with less serious side effects than most traditional chemotherapeutic agents. Chemotherapeutic agents have significant toxic side effects upon repeated long term administration including hair loss, therefore the former class of drugs represent a means of reducing patient discomfort of both a physical and psychological nature.

In addition, the viral agents as hereinbefore described are particularly suitable for use in gene therapy in humans and animals because it is well known that the viruses are susceptible to anti-herpetic drugs such as acyclovir. Thus, in the instance where one wanted to terminate, either temporarily or permanently, a gene therapy treatment, one could simply administer acyclovir to an individual receiving such gene therapy and this would have the effect of destroying the gene therapy vector, and the cells supporting vector replication, thus terminating the therapy. The ability to control the gene therapy treatment once it has commenced is of particular importance in the context of treating children or adults who are capable of reproduction because it enables a gene therapy regime to be terminated before the individual reproduces and it therefore prevents the gene of the relevant treatment being transmitted in the germ cell line and so being passed on through generations. It is also of particular note that it is possible to engineer the gene therapy vector so as to make it particularly sensitive to anti-herpetic drugs such as acyclovir. This can be undertaken using conventional techniques by, for example, the insertion of a herpes simplex virus thymidine kinase gene in the relevant vector. The aforementioned enzyme phosphorylates acyclovir and so renders the virus particularly sensitive to its effects.

The invention claimed in this application is the novel use of animal viruses as arrestable therapeutic agents and optionally the introduction of a heterologous gene which make the virus hypersensitive to treatment with anti-herpetic drugs/agents, thus inventively permitting complete, effective, safe and accurate termination of therapy with minimal side effects to a patient.

It is a first object of the invention to provide an arrestable therapeutic viral agent derived from an animal virus which intrinsically, or following suitable manipulation, is susceptible to anti-herpetic drugs/agents.

It is a further object of the invention to provide an arrestable therapeutic viral agent which comprises a replication-competent virus vector for tumour destruction.

It is a yet further object of the invention to provide an arrestable therapeutic viral agent that is susceptible to elimination from the host/patient using anti-herpetic agents.

It is a yet further object of the invention to minimise the side-effects to the host/patient during cancer therapy treatment.

It is a yet further object of the invention to provide a therapy treatment that can be easily terminated either temporarily or permanently.

According to a first aspect of the invention there is provided use of an arrestable therapeutic viral agent that is susceptible to elimination from a host/patient using anti-herpetic agents and that is capable of replicating in human tissue for treating tumours.

Reference herein to tumours is intended to include any neoplasm, malignant growth, cancer or morbid enlargement in any human tissue of a solid nature or otherwise.

Reference herein to arrestable is intended to include either temporary or permanent arrest of viral activity.

Reference herein to elimination is intended to include short, medium or long term elimination, typically characterised by cessation of virus replication.

Reference herein to anti-herpetic drugs/agents is intended to include reference to any known agent that has been demonstrated to have anti-herpetic activity and is intended to include analogues and homologues thereof.

In a yet further preferred embodiment of the invention there is provided use of a herpetic virus capable of replicating in human tissue ideally for use in treating tumours.

Ideally said herpetic virus is engineered or altered so as to exhibit enhanced sensitivity to said anti-herpetic agents.

Reference herein to herpetic virus is intended to include reference to any virus that is intrinsically sensitive to or suitably altered so as to be sensitive to anti-herpetic drugs/agents such as acyclovir and/or pencyclovir and/or homologues and/or analogues thereof including other related drugs/agents such as those typically known as orphan drugs.

EHV-1 has been chosen as it is one of the few herpesviruses whose natural route of infection is at mucosal surfaces, additionally the virus replicates in cell lines derived from a wide range of human tumours, but does not cause diseases in humans (3). The virus is much more stable in such an environment than other herpesviruses. EHV-1 is not a human pathogen and there is no innate immune response to this virus in the population. Thus the virus would not be inhibited from spreading through a tumour on primary inoculation, but the infected cells would not represent (at least to start with) targets for host destruction.

Both the aforementioned viruses replicate in a wide range of human cell types in culture, destroying their host cell. All herpesviruses are variably susceptible to inhibition by acyclovir. An additional advantage of both viruses is that they maybe engineered to replicate only in dividing cells.

In a yet further preferred embodiment of the invention there is provided an herpes virus capable of replicating in human tissue ideally for use in treating tumours, said herpes simplex virus is HSV-1 strain HFEM.

In a yet further preferred embodiment of the invention there is provided use of an equine herpesvirus capable of replicating in human tissue ideally for use in treating tumours.

Ideally said equine herpesvirus is engineered or altered so as to exhibit enhanced sensitivity to said anti-herpetic agent.

We know, preferably, since the virus gene product responsible for activating acyclovir, TK, is expressed at a lower level in EHV-1 than HSV-1, thus making the virus less susceptible to the antiviral drug, we can replace this gene with that derived from HSV-1 to inventively improve the sensitivity to acyclovir.

In addition we are deleting three genes which are known to be important for EHV-1 pathogenicity of the disease in small animal models. This will ensure that the virus is considered by regulatory authorities to be a severely disabled, replication-competent virus which does not need to be handled under a high level of containment, which might otherwise be a problem when introducing a heterologous gene into an otherwise wild-type virus. Thus we have invented a novel approach to overcoming problems associated with the prior art.

In a yet further preferred embodiment of the invention any of the aforementioned said viruses lacks or has at least one mutation in at least one gene responsible for, or associated with, pathogenicity in humans, such that the gene product is lacking or is non-functional with respect to human pathogenicity.

Thus any of the aforementioned viruses have been genetically manipulated so as to remove genes which are known to be determinants of pathogenicity typically in animal models. The agent derived from HSV-1 is based on a laboratory-adapted strain of the virus which has a spontaneous deletion in the genome and affects a gene responsible for the spread of the virus from peripheral sites (4). Additional mutations which are known to affect pathogenicity will be introduced into this virus.

Optionally, as EHV-1 is apparently apathogenic for humans, there may be no need for gene deletion.

In a yet further preferred embodiment of the invention there is provided an animal virus modified so that at least one gene relating to pathogenicity is/are mutated and/or deleted and/or disabled.

Thus in a yet further preferred embodiment of the invention there is provided an herpesvirus, as hereinbefore described, modified so that at least one gene relating to pathogenicity is/are mutated and/or deleted and/or disabled, ideally said herpesvirus is HSV-1 strain HFEM and most ideally said gene determinant(s) is/are U13 and/or UL50.

Thus is a yet further preferred embodiment of the invention there is provided an equine herpesvirus, as hereinbefore described, modified so that at least one gene relating to pathogenicity is/are mutated and/or deleted and/or disabled, ideally said gene determinant(s) is/are gene 13 and/or gene 49 and/or gene 9.

According to a second aspect of the invention there is provided a modified acyclovir-sensitive animal virus that is replication-competent but has been genetically altered so that it exhibits enhanced sensitivity to acyclovir or homologues or analogues thereof.

In a preferred embodiment of the invention there is provided a modified acyclovir-sensitive herpesvirus that is replication competent but has been genetically altered so that it exhibits enhanced sensitivity to acyclovir or homologues or analogues thereof.

In a yet further preferred embodiment of the invention there is provided a modified equine herpesvirus that is replication-competent but has been genetically altered so that it exhibits enhanced sensitivity to acyclovir or homologues or analogues thereof.

In a yet further preferred embodiment of the invention there is provided an animal virus modified so as to carry a selected gene or agent such as a drug for use in therapy, ideally said animal virus comprises an herpesvirus and more ideally said animal virus comprises an equine herpesvirus.

According to a third aspect of the invention there is provided a pharmaceutical composition comprising either one or more of said aforementioned viruses, optionally in combination with a suitable carrier, ideally said carrier comprises a solution or suspension or the like.

According to a fourth aspect of the invention there is provided a method of treating an individual, wherein said individual is identified as having or likely to have a tumour, comprising:-
I administering the aforementioned pharmaceutical composition to said individual to be treated, ideally the route of administration is by injection and most ideally injection directly to a tumour site and/or its immediate environs; and
II optionally, selectively terminating or abating treatment with said pharmaceutical composition by administering to said individual an effective amount of an anti-viral agent, ideally the route of administration of said anti-viral agent is by injection and/or oral and most ideally said anti-viral agent is an anti-herpetic agent such as acyclovir and/or homologues and/or analogues thereof.

### Methods

### Inactivation of the EHV-1 thymidine kinase gene and production of a new strain of EHV-1 incorporating the thymidine kinase gene from herpes simplex virus type 1.

The EHV-1 TK gene is situated between coordinates 69910 and 70968 in the published sequence of virus strain Ab4. The virus strain to be manipulated was Ab1, isolated at exactly the same time as Ab4 and has an essentially identical genome sequence (5). The DNA sequence between coordinates 69910 and 71200 was amplified by PCR and the 1290bp fragment was cloned into the plasmid LITMUS 38. This plasmid termed pDM551 was digested with the restriction endonuclease Fsp 1 to remove a 315bp fragment of the TK gene coding sequence. The plasmid was then religated to create pDM552. This construct was transfected into BHK cells with purified infectious EHV-1 DNA using standard procedures. Cytopathic effect was allowed to proceed until the majority of the cell sheet was destroyed by infectious virus. Cells were then scraped from the dish, disrupted by ultrasonic vibration and the titre of the virus stock determined, in the presence and absence of 100µg/ml acyclovir, which estimated the percentage of the virus population resistant to acyclovir, i.c. those that were now TK negative. The virus stock was replated using limiting dilution methods in 96 well plates in tissue culture medium containing acyclovir. Individual plaque isolates of acyclovir-resistant viruses were then tested using PCR for the presence of the engineered TK deletion. Stocks of this virus were then plaque purified and designated as Ab1_38.

The HSV-1 TK gene (UL23) is located between nucleotides 46674 and 47802 in the published sequence of HSV-1 strain 17. The complete coding sequence was amplified by PCR and cloned into the pCMVbeta, from which the beta-galactosidase gene had been removed by digestion with the restriction endonuclease Not 1, generating pDM553. An expression cassette consisting of the TK gene under the control of the CMV promoter was removed from this plasmid using restriction endonucleases Eco R1 and Sal 1, and ligated into the Fsp 1 site of pDM552, to create plasmid pDM554. Infectious virus DNA prepared from AB4_38 was cotransfected with DNA from pDM552 and progeny virus isolated and titrated. Individual virus stocks were isolated through plating at limiting dilution, and then replica plated into duplicate 96 well plates, one in the presence of 100µg/ml acyclovir. Viruses which were now susceptible to acyclovir (i.e. were recombinants expressing HSV-1 TK), were removed from the replica plate grown in the absence of acyclovir, and subjected to 2 rounds of further plaque purification. Genome integrity was confirmed by Southern blotting, and the derivative viruses were termed AB1RTK1.

### HSV-1 gene UL13

The UL13 gene is located between coordinates 26950 and 28504 in the published sequence of HSV-1 strain 17. The complete gene was available in this laboratory in a plasmid designated pYW101 which contains a DNA fragment derived from HSV-1 strain 17 between coordinates 29336 and 25149. A fragment of this was derived by digestion with Dde 1 and cloned into the Sma 1 site of pUC19. This plasmid was termed pYW201. Further digestion of this plasmid with Hind III removed a 52bp fragment from the UL13 coding sequence, the plasmid was religated and termed pYW211. This plasmid was then redigested with HindII and a cassette containing the beta-galactoside gene under control of the CMVIE3 promoter was cloned into the plasmid to derive pYW212. Infectious virus DNA was transfected with pYW212 exactly as aforedescribed and cytopathic effect allowed to procede. Progeny virus was isolated, diluted and plated out on fresh monolayers of BHK cells in the presence of the beta-galactoside colourimetric, X-gal. Virus plaques which turned blue were picked and recombinant virus was isolated in a clonal form through 3 rounds of plaque purification. Genome integrity was confirmed by Southern blotting.

### HSV-1 gene UL50

The HSV-1 UL50 gene is located between coordinates 107,010 and 108,123 in the published sequence of HSV-1 strain 17. This DNA sequence as above was amplified by PCR and the 1114 base pair fragment was cloned into plasmid LITMUS 38. The resulting plasmid, designated pDM710, was digested with the restriction endonuclease Sma 1 to release a 96 bp and a 47bp fragment. The remaining plasmid was made blunt ended with T4 DNA polymerase and a cassette of the beta-galactosidase gene under control of the CMVIE3 promoter was ligated into site to create pDM711. Infectious virus DNA was transfected with pDM711 exactly as described in the previous section and cytopathic effect allowed to procede. Progeny virus was isolated, diluted and plated out on fresh monolayers of BHK cells in the presence of the beta- galactosidase colourimetric indicator, X-gal. Virus plaques which turned blue were picked and recombinant virus was isolated in a clonal form through 3 rounds of plaque purification. Genome integrity was confirmed by Southern blotting.

### EHV-1 gene 13

EHV-1 gene 13 is located between coordinates 15317 and 17932 in the published sequence of EHV-1 strain Ab4. This DNA sequence (from strain Ab1 as above was amplified by PCR and the 2640 base pair fragment was cloned into plasmid LITMUS 38. The resulting plasmid, designated pDM570, was digested with the restriction endonucleases Sma 1 and Bam H1 to release a 1333bp fragment. The remaining plasmid was made blunt ended with T4 DNA polymerase and a cassette of the beta-galactosidase gene under control of the CMVIE3 promoter was ligated into the site to create pDM571. Infectious virus DNA was transfected with pDM571 exactly as described in the previous section and cytopathic effect allowed to procede. Progeny virus was isolated, diluted and plated out on fresh monolayers of BHK cells in the presence of the beta-galactosidase colourimetric indicator, X-gal. Virus plaques which turned blue were picked and recombinant virus was isolated in a clonal form through 3 rounds of plaque purification. Genome integrity was confirmed by Southern blotting.

### Deletion of gene 49, virion protein kinase

The gene encoding the virion protein kinase is located between genome coordinates 89369 and 91153. The complete coding sequence was amplified by PCR from purified virus DNA as template to generate a 1782 bpfragment. This was cloned into the plasmid LITMUS38 to create plasmid pDM561. This was then digested with the restriction endonucleases Nru1 and Pml 1 to release a 644bp fragment. The plasmid was then made blunt ended and a cassette of the beta-galactosidase gene under control of the CMV IE3 promoter was ligated into the site to generate pDM562. Infectious virus DNA was co-transfected with plasmid pDM562 into BHK cells, and cytopathic effect allowed to proceed. Progeny virus was harvested, diluted and plated out onto fresh monolayers of BHK cells in the presence of the beta-galactosidase colorimetric indicator, X-gal. Virus plaques which turned blue were picked and the recombinant virus was isolated in a clonal form through several rounds of plaque purification. Genome integrity was confirmed by Southern blotting.

### EVH-1 gene 9

EHV-1 gene 9 is located between coordinates 11,135 and 12,115 in the published sequence of EHV-1 strain Ab4. This DNA sequence (from strain Ab1 as above) was amplified by PCR and the 980 base pair fragment was cloned into the plasmid LITMUS 38. The resulting plasmid, designated pDM580, was designated with the restriction endonucleases Pml 1 and Bsp M1 to release a 377bp fragment. The remaining plasmid was made blunt ended with T4 DNA polymerase and a cassette of the beta-galactosidase gene under control of the CMVIE3 promoter was ligated into the site to create pDM581. Infectious virus DNA was transfected with pDM581 exactly as described in the previous section and cytopathic effect allowed to procede. Progeny virus was isolated, diluted and plated out on fresh monolayers of BHK cells in the presence of the beta galactosidase colourimetric indicator, X-gal. Virus plaques which turned blue were picked and recombinant virus was isolated in a clonal form through 3 rounds of plaque purification. Genome integrity was confirmed by Southern blotting.

### Construction of a Recombination Cassette, to Create a Strain of EHV-1 Containing the HSV-1 TK Gene.

The plasmid pHSV-106 (Life Science Technologies) was digested with BamH1 to produce a 3.4Kbp fragment containing the complete HSV-1 TK gene.

Two sets of PCR primers flanking the EHV-1 TK gene (gene 38) were designed to include appropriate restriction enzyme sites to ligate to either end of the BamH1 TK fragment. The 5' flanking region encompasses 404bp, the 3' flanking region encompassed 1,002bp flanking sequences were ligated to the BamH1 fragment, and cloned into pUC18. Clones were screened for the presence of DNA sequences in the correct orientation, and a typical clone was named p38TK.

Purified p38TK was co-transfected with purified EHV-1 DNA, and recombinant virus was isolated using standard procedures.

### References

- 1: Roth, J.A. et al (1996). Nature Medicine 9 985-991
- 2: Andreansky, S.S. et al (1996). Proc. Natl. Acad. Sci 93 11313-11318
- 3: J. Griffiths, M. Cooper and D. M. Meredith (unpublished)
- 4: Becker, Y. et al (1986) Virology 149, 255-259
- 5: G. Whittaker, D. Elton and D. M. Meredith (unpublished)

## Claims

1. An equine herpes virus that has been recombinantly engineered or altered so as to exhibit enhanced sensitivity to an anti-herpetic agent the virus being capable of replicating in human tissue for use as a pharmaceutical, optionally for use in treating tumours.

2. A virus according to Claim 1 that is susceptible to elimination from a host/patient using an anti-herpetic agent.

3. A virus according to either Claim 1 or 2 wherein said engineering involves the insertion of a herpes simplex virus thymidine kinase gene therein.

4. A virus according to any preceding claim wherein the virus has been engineered or altered so as to exhibit enhanced sensitivity to an anti-herpetic agent whereby said equine herpes virus can be eliminated from a host/patient.

5. A virus according to any preceding claim which replicates only in dividing cells.

6. An virus according to any preceding claim wherein said equine herpes virus is type 1 (EHV-1).

7. A virus according to any preceding claim wherein the virus lacks, or has at least one mutation in, at least one gene responsible for, or associated with, pathogenicity in humans, such that the gene product is lacking or non-functional with respect to human pathogenicity.

8. A virus according to Claim 7 wherein said pathogenic gene is mutated and/or deleted and/or disabled.

9. A virus according to Claims 7 or 8 wherein said pathogenic gene is gene 13 and/or gene 49 and/or gene 9.

10. A virus according to any preceding claims wherein the virus has been modified so as to carry a selected gene or agent such as a drug for use in therapy.

11. A modified, anti-herpetic sensitive, equine herpes virus that is replication-competent but that has been genetically altered so that it exhibits enhanced sensitivity to anti-herpetic agents or homologues or analogues thereof.

12. A virus according to Claim 11 for use as a pharmaceutical.

13. A virus according to either Claims 11 or 12 which further includes any of the feature(s) of Claims 2-10.

14. A pharmaceutical composition comprising the virus of Claims 1-10 or the virus of Claims 11-13 and optionally a suitable carrier.

15. A herpes simplex virus HSV-1 strain HFEM virus capable of replicating in human tissue for use as a pharmaceutical, optionally for use in treating tumours.

16. A virus according to Claim 15 that is susceptible to elimination from a host/patient using an anti-herpetic agent.

17. A virus according to any of Claims 14-16 wherein said virus has been recombinantly engineered or altered so as to exhibit enhanced sensitivity to said anti-herpetic agent.

18. A virus according to Claim 17 wherein said engineering involves the insertion of a herpes simplex virus thymidine kinase gene therein.

19. A virus according to any of Claims 14-18 wherein the virus has been engineered or altered so as to exhibit enhanced sensitivity to an anti-herpetic agent whereby said equine herpes virus can be eliminated from a host/patient.

20. A virus according to any of Claims 14-19 which replicates only in dividing cells.

21. A virus according to any of Claims 14-20 wherein the virus lacks, or has at least one mutation in, at least one gene responsible for, or associated with, pathogenicity in humans, such that the gene product is lacking or non-functional with respect to human pathogenicity.

22. A virus according to Claim 21 wherein said pathogenic gene is mutated and/or deleted and/or disabled.

23. A virus according to Claims 21 or 22 wherein said pathogenic gene is U13 and/or U50.

24. A virus according to any one of Claims 14-23 wherein the virus has been modified so as to carry a selected gene or agent such as a drug for use in therapy.

25. A modified, anti-herpetic sensitive, herpes simplex virus HSV-1 strain HFEM virus that is replication-competent but that has been genetically altered so that it exhibits enhanced sensitivity to anti-herpetic agents or homologues or analogues thereof.

26. A virus according to Claim 25 for use as a pharmaceutical.

27. A virus according to either of Claims 25 or 26 which further includes any of the feature(s) of Claims 16-24.

28. A pharmaceutical composition comprising the virus of Claims 15-24 or the virus of Claims 25-27 and optionally a suitable carrier.

29. Use of a virus according to any of Claims 1 or 11 or 15 or 25 for the manufacture of a medicament in the treatment of cancer.

30. Use according to claim 29 further comprising selectively terminating or abating treatment by administration of a therapeutically effective amount of an anti-herpetic agent.

31. Use according to claim 30 wherein said anti-herpetic agent is acyclovir.

## Patentansprüche

1. Pferde-Herpes-Virus das gentechnisch rekombinant hergestellt oder verändert wurde, um gesteigerte Empfindlich gegen einen anti-herpetischen Wirkstoff zu zeigen, wobei das. Virus in der Lage ist, sich in menschlichem Gewebe zu replizieren, für die Verwendung als Pharmazeutikum, optional für die Verwendung bei der Behandlung von Tumoren.

2. Virus gemäß Anspruch 1, das empfindlich ist für die Eliminierung aus Wirten/Patienten, bei Verwendung eines anti-herpetischen Wirkstoffes.

3. Virus gemäß Anspruch 1 oder 2, wobei diese gentechnische Herstellung die Insertion eines Herpes-Simplex-Virus-Thymidin-Kinase-Gens in das Virus einschließt.

4. Virus gemäß irgendeinem der vorstehenden Ansprüche, wobei das Virus gentechnisch hergestellt oder verändert wurde, um gesteigerte Empfindlichkeit gegen einen anti-herpetischen Wirkstoff zu zeigen und wobei dieses Pferde-Herpes-Virus aus dem Wirt/Patienten eliminiert werden kann.

5. Virus gemäß einem der vorstehenden Ansprüche, das sich nur durch Zellteilung repliziert.

6. Virus gemäß einem der vorstehenden Ansprüche, wobei das Pferde-Herpes-Virus vom Typ 1 (EHV-1) ist.

7. Virus gemäß einem der vorstehenden Ansprüche, wobei dem Virus ein Gen fehlt oder es mindestens eine Mutation in einem Gen besitzt, das verantwortlich ist für oder in Zusammenhang steht mit der Pathogenizität bei Menschen, so dass dem Genprodukt diese fehlt oder in Bezug auf menschliche Pathogenizität nicht funktionsfähig ist.

8. Virus gemäß Anspruch 7, bei dem dieses pathogene Gen mutiert ist und/oder deletiert und/oder unwirksam ist.

9. Virus gemäß den Ansprüchen 7 oder 8, worin dieses pathogene Gen Gen 13 und/oder Gen 49 und/oder Gen 9 ist.

10. Virus gemäß einem der vorstehenden Ansprüche, worin das Virus modifiziert wurde, um ein ausgewähltes Gen oder einen Wirkstoff, wie ein Arzneimittel zur Verwendung in der Therapie zu tragen.

11. Modifiziertes, anti-herpetisch-empfindliches, Pferde-Virus, das replikationsfähig ist, das aber genetisch verändert wurde, so dass es gesteigerte Empfindlichkeit gegen anti-herpetische Wirkstoffe oder deren Homologe oder deren Analoge zeigt.

12. Virus gemäß Anspruch 11, zur Verwendung als Pharmazeutikum.

13. Virus gemäß den Ansprüchen 11 oder 12, das weiterhin irgendein Merkmal oder mehrere der Merkmale der Ansprüche 2-10 einschließt.

14. Pharmazeutische Zusammensetzung, die das Virus der Ansprüche 1-10 oder das Virus der Ansprüche 11-13 und optional einen geeigneten Träger umfasst.

15. Herpes-Simplex-Virus HSV-1-Stamm HFEM-Virus, zur Replikation in menschlichem Gewebe befähigt, zur Verwendung als Pharmazeutikum, optional zur Verwendung bei der Tumorbehandlung.

16. Virus gemäß Anspruch 15, das durch Anwendung eines anti-herpetischen Wirkstoffs zugänglich ist für die Eliminierung aus einem Wirt/Patienten.

17. Virus gemäß einem der Ansprüche 14-16, worin dieses Virus gentechnisch hergestellt oder verändert wurde, um gesteigerte Empfindlichkeit gegen diesen anti-herpetischen Wirkstoff zu zeigen.

18. Virus gemäß Anspruch 17, worin diese gentechnische Herstellung das Insertieren eines Herpes-Simplex-Virus-Thymidin-Kinase-Gens in das Virus umfasst.

19. Virus gemäß einem der Ansprüche 14-18, worin das Virus gentechnisch hergestellt oder verändert wurde, um gesteigerte Empfindlichkeit gegen einen anti-herpetischen Wirkstoff zu zeigen, wodurch dieses Pferde-Herpes-Virus aus dem Wirt/Patienten eliminiert werden kann.

20. Virus gemäß einem der Ansprüche 14-19, das sich nur durch Zellteilung repliziert.

21. Virus gemäß einem Ansprüche 14-20, wobei dem Virus ein Gen fehlt oder es mindestens eine Mutation in einem Gen besitzt, das verantwortlich ist für oder in Zusammenhang steht mit der Pathogenizität bei Menschen, so dass dem Genprodukt diese fehlt oder in Bezug auf menschliche Pathogenizität nicht funktionsfähig ist.

22. Virus gemäß Anspruch 21, bei dem dieses pathogene Gen mutiert ist und/oder deletiert und/oder unwirksam ist.

23. Virus gemäß den Ansprüchen 21 oder 22, worin dieses pathogene Gen U13 und/oder U50 ist.

24. Virus gemäß einem der Ansprüche 14-23, worin das Virus modifiziert wurde, um ein ausgewähltes Gen oder einen Wirkstoff, wie ein Arzneimittel, zur Verwendung in der Therapie, zu tragen.

25. Modifiziertes, anti-herpetisch-empfindliches, Herpes-Simplex-Virus HSV-1 Stamm HFEM-Virus, das replikationsfähig ist, aber das genetisch verändert wurde, um gesteigerte Empfindlichkeit gegen anti-herpetische Wirkstoffe, deren Homologe oder Analoge zu zeigen.

26. Virus gemäß Anspruch 25, zur Verwendung als Pharmazeutikum.

27. Virus gemäß den Ansprüchen 25 oder 26, das weiterhin irgendein Merkmal oder mehrer der Merkmale der Ansprüche 16-24 einschließt.

28. Pharmazeutische Zusammensetzung, die das Virus der Ansprüche 15-24 oder das Virus der Ansprüche 25-27 und optional einen geeigneten Träger umfasst.

29. Verwendung eines Virus gemäß irgendeinem der Ansprüche 1 oder 11 oder 15 oder 25 zur Herstellung eines Medikaments zur Behandlung von Krebs.

30. Verwendung gemäß Anspruch 29, die weiterhin die selektive Beendigung oder den Abbruch der Behandlung durch Verabreichung einer therapeutisch wirksamen Menge eines anti-herpetischen Wirkstoffs umfasst.

31. Verwendung gemäß Anspruch 30, worin dieser anti-herpetische Wirkstoff Acyclovir ist.

## Revendications

1. Un herpès équine virus qui a été travaillé de manière recombinatoire ou altéré de façon à montrer une sensibilité améliorée à un agent anti herpès, le virus étant capable de réplication dans le tissu humain pour utilisation pharmaceutique, optionnellement pour une utilisation destinée au traitement des tumeurs.

2. Virus selon la revendication 1, **caractérisé en ce qu'**il est susceptible d'élimination à partir d'un hôte / patient en utilisant un agent anti herpès.

3. Virus selon l'une des revendications 1 ou 2, **caractérisé en ce que** ledit traitement implique l'insertion d'un gène kinase thymidine virus simplex herpès à l'intérieur.

4. Virus selon l'une des revendications précédentes, **caractérisé en ce qu'**il a été traité ou altéré de manière à montrer une sensibilité améliorée à un agent anti herpès tandis que ledit virus herpès équine peut être éliminé d'un hôte / patient.

5. Virus selon l'une des revendications précédentes, **caractérisé en ce qu'**il se réplique seulement en division de cellules.

6. Virus selon l'une des revendications précédentes, **caractérisé en ce que** ledit virus herpès équine est du type 1 (EHV-1).

7. Virus selon l'une des revendications précédentes, **caractérisé en ce qu'**il lui manque, ou présente au moins une mutation dans, un gène responsable de, ou associé à, un caractère pathogénique chez les humains, de façon que le produit de gène manque ou n'est pas fonctionnel par rapport au caractère pathogénique humain.

8. Virus selon la revendication 7, **caractérisé en ce que** ledit gène pathogénique subit une mutation et/ou est supprimé et/ou est désactivé.

9. Virus selon l'une des revendications précédentes, **caractérisé en ce que** ledit gène pathogénique est gène 13 et/ou gène 49 et/ou gène 9.

10. Virus selon l'une des revendications précédentes, **caractérisé en ce qu'**il a été modifié afin de porter un gène choisi ou un agent tel qu'un médicament pour utilisation en thérapie.

11. Virus herpès équine modifié, sensible anti herpès, qui est capable de réplication mais qui a été altéré de manière génétique de façon qu'il montre une sensibilité augmentée à des agents anti herpès ou des homologues ou analogues de ces derniers.

12. Virus selon la revendication 11, destiné à l'utilisation pharmaceutique.

13. Virus selon l'une des revendications 11 ou 12, **caractérisé en ce qu'**il comporte en outre une quelconque de la ou des caractéristique(s) des revendications 2 à 10.

14. Composition pharmaceutique comprenant le virus des revendications 1 à 10 ou le virus des revendications 11 à 13 et optionnellement un porteur adapté.

15. Un virus HFEM ..strain...HSV-1 virus simplex herpès capable de réplication dans le tissu humain pour utilisation pharmaceutique, optionnellement pour utilisation pour le traitement des tumeurs.

16. Virus selon la revendication 15, **caractérisé en ce qu'**il est susceptible d'élimination d'un hôte / patient utilisant un agent anti herpès.

17. Virus selon l'une des revendications 14 à 16, **caractérisé en ce qu'**il est traité de manière recombinatoire ou altérée de manière à montrer une capacité augmentée audit agent anti herpès.

18. Virus selon la revendication 17, **caractérisé en ce que** ledit traitement implique l'insertion d'un gène kinase thymidine virus simplex herpès à l'intérieur.

19. Virus selon l'une des revendications 14 0 18, **caractérisé en ce qu'**il a été travaillé ou altéré de manière à montrer une capacité augmentée à un agent anti herpès tandis que ledit virus herpès équine a été éliminé de l'hôte / patient.

20. Virus selon l'une des revendications 14 à 19, **caractérisé en ce qu'**il se réplique seulement par division de cellules.

21. Virus selon l'une des revendications 14 à 20, **caractérisé en ce qu'**il lui manque, ou présente au moins une mutation dans, au moins un gène responsable pour, ou associé à, un caractère pathogénique chez l'homme, de façon que le produit de gène manque ou n'est pas fonctionnel par rapport au caractère pathogénique humain.

22. Virus selon la revendication 21, **caractérisé en ce que** ledit gène pathogénique est soumis à mutation et/ou supprimé et/ou désactivé.

23. Virus selon l'une des revendications 21 ou 22, **caractérisé en ce que** ledit gène pathogène est U13 et/ou U50.

24. Virus selon l'une des revendications 14 à 23, **caractérisé en ce que** le virus a été modifié de manière à porter un gène choisi ou un agent tel qu'une drogue pour utilisation en thérapie.

25. Un virus HFEM à contrainte virus HSV-1 simplex herpès, sensible anti herpès, qui est capable de réplication, mais a été génétiquement modifié de façon qu'il montre une capacité augmentée à des agents anti herpès ou homologues ou analogues de ces derniers.

26. Virus selon la revendication 25, destinée à l'utilisation pharmaceutique.

27. Virus selon l'une des revendications 25 ou 26, et incluant l'une des caractéristiques des revendications 16 à 24.

28. Composition pharmaceutique comprenant le virus des revendications 15 à 24 ou le virus des revendications 25 à 27 et optionnellement un porteur adapté.

29. Utilisation d'un virus selon l'une des revendications 1 ou 11 ou 15 ou 25, pour la fabrication d'un médicament dans le traitement du cancer.

30. Utilisation selon la revendication 29, **caractérisé en ce qu'**elle comporte en outre l'étape de sélectivement terminer ou diminuer le traitement par administration d'une quantité effective sur le plan thérapeutique d'un agent anti herpès.

31. Utilisation selon la revendication 30, **caractérisé en ce que** ledit agent anti herpès est acyclovir.
